# FASCICULE DE BREVET EUROPEEN

(11) **EP 3 509 660 B1**
(45) Date de publication et mention de la délivrance du brevet: **11.12.2024**
(21) Numéro de dépôt: 17768497.4
(22) Date de dépôt: 05.09.2017
(51) Int. Cl.: A61M 1/02, A61M 1/36

(54) **UNITÉ DE FILTRATION COMPRENANT UN BORD PÉRIPHÉRIQUE COURBE**
FILTRATIONSEINHEIT MIT EINER GEKRÜMMTEN RANDKANTE
FILTRATION UNIT COMPRISING A CURVED PERIPHERAL EDGE

(30) Priorité: 08.09.2016 FR 1658379
(43) Date de publication de la demande: 17.07.2019
(73) Titulaire: Maco Pharma, 59420 Mouvaux (FR)
(72) Inventeur: BREBANT, Quentin, 59110 La Madeleine (FR); DEKERKE, Damien, 59710 Pont-à Marc (FR)
(74) Mandataire: Sayettat, Julien Christian
(86) Numéro de dépôt international: PCT/FR2017/052353
(87) Numéro de publication internationale: WO 2018/046843

(56) Documents cités:
- EP-A1- 2 878 316
- EP-A2- 1 156 067
- US-A1- 2012 160 782

## Description

L'invention concerne une unité de filtration d'un fluide biologique tel que le sang ou un composant sanguin ainsi qu'un système à poches comprenant une telle unité de filtration.

L'invention s'applique au domaine de la filtration des fluides biologiques et notamment la filtration du sang ou des composants sanguins.

Dans le champ de la transfusion, il est classique de traiter le sang pour en éliminer les substances indésirables telles que les leucocytes, les pathogènes, certaines protéines - par exemple le prion -, et/ou les substances utilisées dans des procédés d'inactivation, de réduction et/ou d'élimination des pathogènes.

Pour éliminer ces substances indésirables par filtration, il existe deux grandes catégories d'unités de filtration : les unités de filtration dites souples comprenant une enveloppe extérieure souple, et les unités de filtration dites rigides comprenant un boîtier rigide. Un milieu de filtration apte à éliminer les substances indésirables du fluide est enfermé dans ces unités de filtration.

Ces unités de filtration sont généralement intégrées dans des systèmes à poches permettant de traiter le sang et les composants sanguins en circuit clos.

Ainsi, après recueil du sang total, de tels systèmes à poches sont placés dans des appareils de centrifugation dans le but de séparer les différents composants sanguins en fonction de leur densité, en les soumettant à la force centrifuge.

Cependant, durant cette étape de centrifugation, il existe un risque non seulement que ces unités de filtration rigides soient endommagées mais encore que les unités de filtration rigides endommagent les poches adjacentes, rendant inutilisables les systèmes à poches et contaminant possiblement l'environnement extérieur et l'utilisateur.

En effet, comme illustré dans le document WO 2008/103142, les unités de filtration rigides comprennent des boîtiers aux bords formant des angles sensiblement droits et possèdent en outre des orifices d'entrée et/ou sortie de fluide faisant saillie du boîtier en formant des arêtes vives. Avec la pression générée par la centrifugation, ces parties anguleuses peuvent perforer les poches.

Même si certaines unités de filtration rigides possèdent des coins arrondis, comme celles illustrées dans le document EP 1 156 067 A2, ces unités comportent toujours des parties anguleuses risquant d'endommager les poches souples lors de la centrifugation.

Pour éviter la dégradation des systèmes à poches durant la centrifugation, il a donc été développé des unités de filtration de type souple, par exemple comme celle du document EP-A-526 678. Ces unités de filtration souples sont cependant difficiles à fabriquer.

D'autre part, différentes solutions ont été proposées dans le but d'éviter les dommages liés aux unités de filtration rigides au cours de la centrifugation.

Par exemple, dans le document EP 0627 228, il est proposé de placer les unités de filtration en dehors du godet de centrifugation sur le rotor de la centrifugeuse.

Dans la même veine, dans le document US 5 100 564, l'unité de filtration est disposée au-dessus du godet de centrifugation à l'aide d'un support approprié.

D'autre part, il est connu du document EP 0 679 490 A2, un procédé pour fabriquer une unité de filtration rigide comprenant l'alignement d'un milieu de filtration entre deux éléments de boîtier et le moulage par injection d'une bande surmoulée thermoplastique rigide autour des deux éléments de boîtier de sorte à former un joint hermétique à l'extrémité du milieu de filtration. L'unité de filtration obtenue comprend des parties saillantes et anguleuses inadaptées pour la centrifugation avec des poches souples.

L'invention propose une unité de filtration de type essentiellement rigide qui peut être centrifugée dans un godet de centrifugation avec des poches souples sans les endommager.

Ainsi, et selon le premier aspect, l'invention propose une unité de filtration destinée à la filtration d'un fluide comprenant un boîtier formé d'un premier élément de boîtier et d'un deuxième élément de boîtier entre lesquels un milieu de filtration disposé de sorte à définir un compartiment d'entrée pour le fluide à filtrer et un compartiment de sortie pour le fluide filtré, ledit boîtier comportant un conduit d'entrée débouchant dans le compartiment d'entrée et un conduit de sortie débouchant dans le compartiment de sortie, chacun desdits éléments de boîtier comprenant un fond s'étendant dans un plan médian en étant entouré par une périphérie, lesdits éléments de boîtier étant assemblés entre eux de façon étanche au niveau de leur périphérie, le boîtier présentant un bord périphérique ayant un profil extérieur courbe qui s'étend entre les plans médians des fonds.

Selon un deuxième aspect, l'invention concerne un système à poches pour la filtration d'un fluide, comprenant :
- une unité de filtration selon le premier aspect de l'invention, et
- une poche de recueil du filtrat, ladite poche de recueil étant reliée, par l'intermédiaire d'une première tubulure, au conduit de sortie de l'unité de filtration.

D'autres objets et avantages apparaîtront au cours de la description qui suit en lien avec les figures annexées selon lesquelles :
La figure 1 représente une vue schématique en perspective de l'unité de filtration selon une réalisation de l'invention.
Les figures 2 à 4 représentent des vues schématiques de deux profils différents de l'unité de filtration de la figure 1.
La figure 5 représente une vue schématique de dessus de l'unité de filtration de la figure 1.
La figure 6 représente une vue schématique en coupe de l'unité de filtration de la figure 1.
Les figures 7a et 7b représentent une vue schématique partielle et en coupe d'une unité de filtration selon deux autres réalisations.
La figure 8 représente une vue schématique en éclaté et de profil des premier et deuxième éléments de boîtier et du milieu de filtration de l'unité de filtration de la figure 1.
La figure 9 représente une vue schématique d'un système à poches comprenant l'unité de filtration de la figure 1.

L'invention concerne une unité de filtration destinée à la filtration d'un fluide, notamment du sang ou d'un composant sanguin tel qu'un plasma y compris un plasma riche en plaquettes et un plasma pauvre en plaquettes, un concentré de globules rouges ou un concentré de plaquettes sanguines.

L'unité de filtration est destinée à éliminer des composants cibles du fluide, notamment les leucocytes, les protéines prions ou les substances d'inactivation des pathogènes tel que le bleu de méthylène.

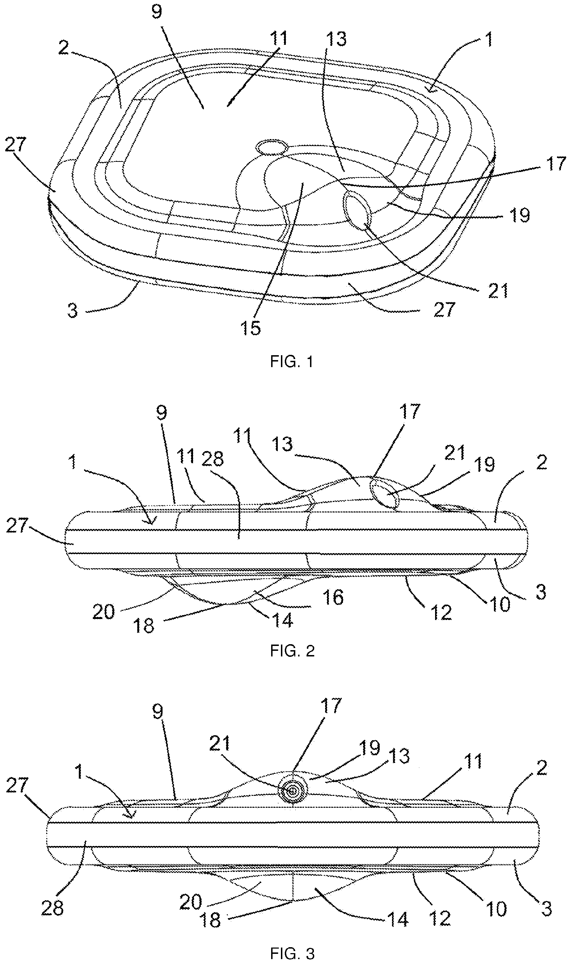

En relation avec les figures 1 à 8, l'unité de filtration 1 comprend un boîtier rigide formé d'un premier élément de boîtier 2 et d'un deuxième élément de boîtier 3 entre lesquels un milieu de filtration 4 est disposé de sorte à définir un compartiment d'entrée 5 pour le fluide à filtrer et un compartiment de sortie 6 pour le fluide filtré.

Les éléments de boîtier sont notamment réalisés dans un matériau polymère thermoplastique tel que le polycarbonate, polychlorure de vinyle, polypropylène ou acrylonitrile-butadiène-styrène.

Le compartiment d'entrée 5 est limité par le premier élément de boîtier 2 et le milieu de filtration 4. Le compartiment de sortie 6 est limité par le deuxième élément de boîtier 3 et le milieu de filtration 4.

Cette unité de filtration 1 est destinée à filtrer un fluide biologique qui est, par exemple un fluide biologique tel que le sang ou un composant sanguin.

Dans une réalisation, le milieu de filtration 4 est apte à éliminer les leucocytes du sang ou d'un composant sanguin. Le milieu de filtration comprend notamment une ou plusieurs couches d'un matériau tissé ou non-tissé. Par exemple, le milieu de filtration comprend un assemblage de plusieurs couches de non-tissé.

Le ou les matériau(x) formant la ou les couche(s) du milieu de déleucocytation sont choisi(s) dans le groupe comprenant les polymères ou les copolymères à base de polypropylène, de polyester, de polyamide, de polyéthylène haute ou basse densité, de polyuréthanne, de fluorure de polyvinylidène, de polyvinylpyrrolidone et leurs dérivés. Avantageusement, le matériau est du polypropylène, du polyéthylène téréphtalate ou du polybutylène téréphtalate.

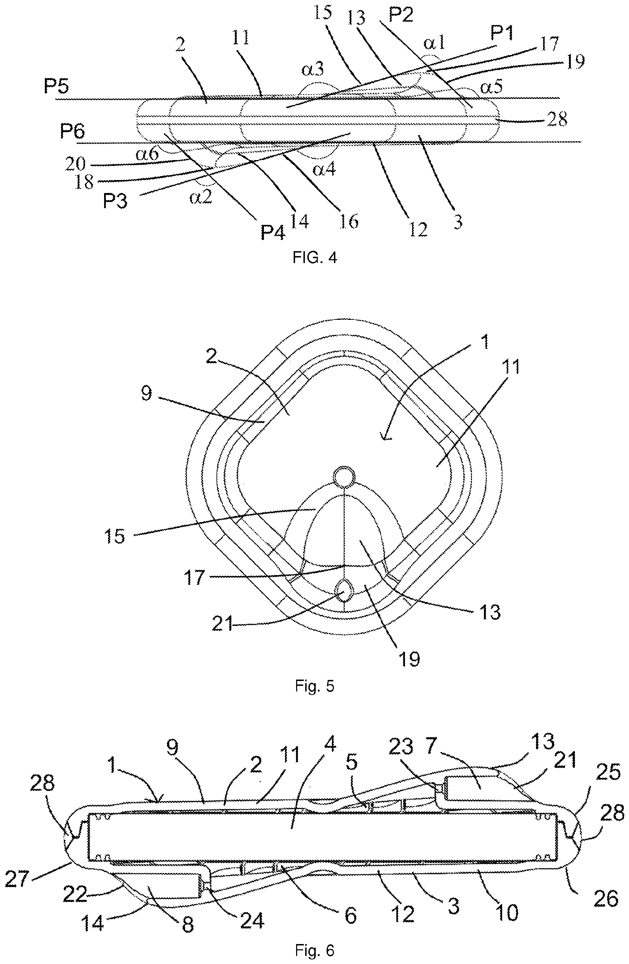

En relation avec la figure 6, le boîtier comporte un conduit d'entrée 7 débouchant dans le compartiment d'entrée 5 et un conduit de sortie 8 débouchant dans le compartiment de sortie 6.

Selon une réalisation, le conduit d'entrée 7 et le conduit de sortie 8 sont agencés parallèlement audit milieu de filtration 4.

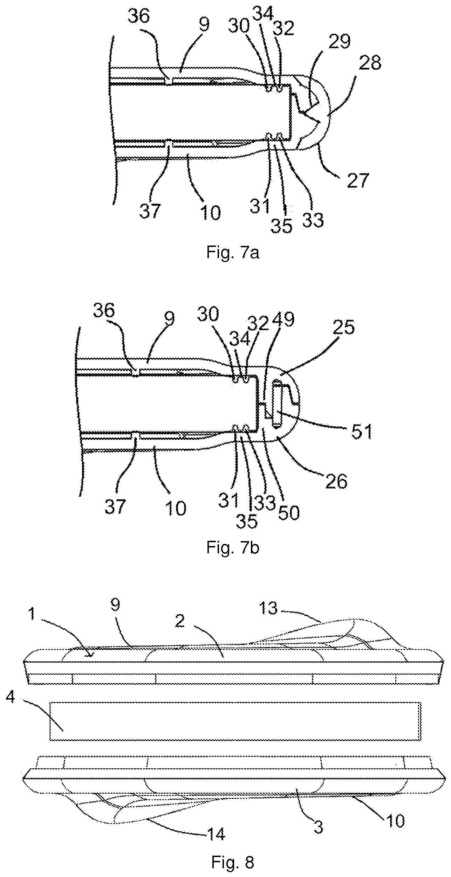

Les conduits d'entrée et sortie 7,8 sont notamment des conduits tubulaires creux et rigides. Ces conduits sont aptes à recevoir une tubulure souple pour pouvoir intégrer l'unité de filtration dans un système à poches tel que celui représenté sur la figure 8.

Dans le mode de réalisation représenté, le premier élément de boîtier 2 comprend le conduit d'entrée 7 pour le fluide à filtrer et le deuxième élément de boîtier 3 comprend le conduit de sortie 8 pour le filtrat.

Comme représenté sur les figures, chacun des premier et deuxième éléments 2,3 de boîtier comprend un fond 9,10 entouré par une paroi périphérique 25, 26, le fond d'au moins un desdits éléments de boîtier comprenant une portion principale 11,12 et au moins une portion en dôme 13,14 qui est délimitée par un pourtour en présentant une paroi interne 15,16 s'étendant depuis ledit pourtour vers un apex 17,18 et une paroi externe 19,20 s'étendant depuis ledit apex 17,18 vers ledit pourtour.

Dans la description et en relation avec l'unité de filtration, l'adjectif "interne" est à comprendre par opposition à l'adjectif "externe". Un moyen dit "externe" désigne un moyen qui est tourné à l'opposé de la partie centrale de l'unité de filtration.

Le fond 9,10 de chacun des éléments de boîtier s'étend dans un plan médian P5,P6 en étant entouré par une paroi périphérique 25, 26.

La portion principale 11,12 du fond des éléments de boîtier 2,3 est notamment sensiblement plane et définit un plan de base.

La portion en dôme 13,14 du fond des éléments de boîtier 2,3 ne comprend avantageusement aucune partie anguleuse, de sorte que le risque de perforer des poches adjacentes lors de la centrifugation est réduit.

Notamment et selon la figure 4, les parois interne 15 et externe 19 de la portion en dôme 13 de l'un des éléments de boîtier 2 s'étendent respectivement dans un plan médian P1,P2 formant entre eux un angle α1 strictement supérieur à 90°, particulièrement compris entre 100° et 130°.

Les parois interne 16 et externe 20 de la portion en dôme 14 de l'autre élément de boîtier 3 s'étendent respectivement dans un plan médian P3,P4 formant entre eux un angle α2 strictement supérieur à 90°, particulièrement compris entre 100° et 130°.

Par « plan médian » d'une paroi, on entend le plan situé au milieu de ladite paroi.

En outre, l'apex 17,18 de la portion en dôme 13,14 présente un profil extérieur courbe qui s'étend entre lesdits plans médians P1 ,P2 e P3,P4 à la jonction entre les parois interne 15,16 et externe 19,20. Le contour extérieur de la portion en dôme 13,14 ne présente ainsi pas de partie droite ou plane.

Selon une réalisation, le profil extérieur courbe possède, vue de profil, un rayon de courbure strictement supérieure à la hauteur de la portion en dôme prise à partir du plan de base P5,P6 de la portion principale 11,12 du fond de l'élément de boîtier 2,3.

Comme illustré sur la figure 4, le plan médian de la paroi interne P1,P3 de la portion en dôme 13,14 forme un angle α3,α4 compris entre 120° et 150° avec le plan de base P5,P6 de la portion principale 11,12 du fond.

Le plan médian P2,P4 de la paroi externe 19,20 de la portion en dôme 13,14 forme un angle α5,α6 compris entre 120° et 150° avec un plan de base P5,P6 de la portion principale 11,12 du fond 9,10.

Ainsi, la portion en dôme 13,14 est relativement peu élevée de sorte que le volume mort du boîtier de l'unité de filtration 1 est réduit.

Plus particulièrement et en relation avec la figure 5, la paroi externe 19,20 s'étend depuis l'apex 17,18 vers une portion du pourtour qui est adjacente à la paroi périphérique 25, 26 du fond 9,10.

La paroi interne 15,16 s'étend vers l'apex 17,18 depuis une portion sensiblement en U du pourtour. Encore plus particulièrement, les extrémités des branches du U sont adjacentes à la paroi périphérique 25, 26 du fond 9,10.

Autrement dit, la portion en dôme 13,14 de l'élément de boîtier 2,3 est située au voisinage de la paroi périphérique 25, 26 du fond 9, 10 de l'élément de boîtier 2,3.

Selon une réalisation particulière, le fond 9,10 présente une dimension qui s'étend le long de la portion principale 11,12 et de la portion en dôme 13,14, la paroi interne 15,16 s'étendant le long de ladite dimension sur une distance comprise entre 20% et 40% de ladite dimension.

Comme représenté sur la figure 6, au moins un des conduits d'entrée 7 et sortie 8 débouche au travers de la paroi externe 19,20 de la portion en dôme 13,14 d'un élément de boîtier 2,3.

Plus particulièrement, une extrémité externe d'un conduit d'entrée 7 et sortie 8 débouche dans la paroi externe 19,20 en étant adjacente à la paroi périphérique 25, 26 du fond 9,10 de l'élément de boîtier 2,3.

Avantageusement, la paroi externe 19,20 présente un orifice dans lequel au moins une extrémité externe 21,22 d'un conduit d'entrée 7 et sortie 8 est inscrite. La portion en dôme 13,14 recouvre alors entièrement le conduit d'entrée 7 ou sortie 8, de sorte qu'aucun élément saillant fragile ne s'étend de la portion en dôme 13,14.

Les parties en relief du boîtier présentent ainsi une forme courbe de sorte que, quelle que soit la position du boîtier dans le godet de centrifugation, le risque de rupture des conduits d'entrée et de sortie 7,8 et le risque d'endommager les poches avoisinantes sont réduits et même éliminés.

Et, dans cette réalisation, l'extrémité externe 21,22 du conduit d'entrée et/ou sortie 7,8 n'est pas droite, c'est-à-dire qu'elle n'est pas perpendiculaire à l'axe longitudinal du conduit d'entrée et/ou sortie 7,8.

Par exemple, comme représenté sur la figure 5, l'extrémité externe du conduit d'entrée et/ou sortie 7,8 présente un biseau. Dans le cas d'une conduite tubulaire, l'orifice est alors sensiblement ovale.

Selon une réalisation particulière représentée sur la figure 6, l'un au moins des conduits d'entrée et de sortie 7,8 présente à son extrémité interne 23,24 une ouverture ayant une section plus petite que la section de l'ouverture dudit conduit 7,8 à son extrémité externe 21,22 (figure 6).

Afin de permettre une meilleure vidange de l'unité de filtration 1, l'extrémité interne 24 du conduit de sortie 8 est pourvue d'une restriction de flux formée par une réduction de section par rapport à la section de ce conduit à son extrémité externe. Par effet Venturi, la restriction de flux créé une accélération du débit de fluide filtré qui tend à aspirer le fluide en dehors de l'unité de filtration 1.

En particulier, l'extrémité interne 23 du conduit d'entrée 7 est également pourvue d'une restriction de flux. Encore plus particulièrement, le conduit d'entrée 7 et le conduit de sortie 8 présentent sensiblement la même géométrie et les mêmes dimensions.

Avantageusement, chacun des éléments de boîtier 2,3 présente une portion en dôme 13, le conduit d'entrée 7 débouchant dans la paroi externe 19 de la portion en dôme 13 dudit premier élément de boîtier 2 et le conduit de sortie 8 débouchant dans la paroi externe 20 de la portion en dôme 14 dudit deuxième élément de boîtier 3.

Selon une réalisation, la portion en dôme 13 du premier élément de boîtier 2 et la portion en dôme 14 du deuxième élément de boîtier 3 présentent sensiblement la même géométrie et les mêmes dimensions.

En relation avec les figures 6, 7a et 7b, chacun des premier et deuxième éléments de boîtier 2,3 comprend une cavité formée du fond 9,10 et d'une paroi latérale périphérique 25,26, la portion en dôme 13,14 étant agencée sur la surface extérieure du fond 9,10 de l'un des premier ou deuxième éléments de boîtier 2,3.

Les éléments de boîtier 2,3 sont assemblés entre eux de façon étanche au niveau de leur périphérie, de sorte à former un boîtier étanche au fluide.

Par exemple, les premier et deuxième éléments de boîtier 2,3 sont assemblés entre eux par soudure par haute fréquence, par radiofréquence ou par ultrasons. En variante, les éléments de boîtier 2,3 sont assemblés par collage.

Afin de faciliter l'assemblage des deux éléments de boîtier 2,3, les éléments de boîtier 2,3 sont ajustés entre eux par assemblage de forme ou par encliquetage.

En relation avec les figures 6, 7a et 7b, la paroi latérale périphérique 25 du premier élément de boîtier 2 et la paroi latérale périphérique 26 du deuxième élément de boîtier 3 sont mises en contact sur leur surface extrême par conjugaison de forme.

Dans une réalisation, le boîtier de l'unité de filtration 1 présente un bord périphérique 27 ayant un profil extérieur courbe qui s'étend entre les plans médians P5,P6 des fonds 9,10 du premier et deuxième élément de boîtier 2,3.

Le contour extérieur du bord périphérique 27, c'est-à-dire la portion joignant les portions substantiellement planes des fonds 9,10 du premier et deuxième élément de boîtier 2,3, ne présente ainsi pas de partie droite ou plane.

Ce bord périphérique 27 au profil extérieur courbe contribue à réduire le risque de dommages aux poches souples lorsque l'unité de filtration et les poches souples sont centrifugées dans un même godet.

Le profil du bord périphérique 27 ne présente substantiellement pas de partie plane.

Notamment, le profil extérieur courbe du bord périphérique 27 présente un rayon de courbure qui est supérieur à la moitié de la distance entre les deux plans médians P5,P6.

Le rayon de courbure est le rayon du cercle tangent au profil extérieur courbe du bord périphérique vu en section transversale.

Selon une première réalisation représentée notamment sur les figures 6 et 7a, l'unité de filtration comprend un élément 28 qui est rapporté sur l'assemblage des périphéries des éléments de boîtier 2,3, le bord périphérique 27 ayant un profil extérieur courbe étant formé sur ledit élément rapporté 28.

De plus, l'élément rapporté 28 est agencé pour assurer une étanchéité de l'assemblage des périphéries des éléments de boîtier 2,3.

En particulier, l'élément rapporté 28 est associé autour des périphéries des éléments de boîtiers 2,3.

Encore plus particulièrement, l'élément 28 est rapporté par surmoulage sur l'assemblage des périphéries des éléments de boîtier 2,3.

Le surmoulage consiste à mettre en place, dans l'empreinte d'une cavité d'un moule d'injection, le boîtier de l'unité de filtration 1, et à injecter l'élément rapporté 28. Le remplissage de l'empreinte permet de réaliser le surmoulage du boîtier avec l'élément rapporté 28.

Par cette technique de surmoulage, l'élément rapporté 28 adhère aux éléments de boîtier 2,3 par liaison physico chimique, le boîtier ne laissant ainsi pas passer le fluide à l'extérieur.

Ainsi, l'assemblage du boîtier est réalisé de manière automatique au moment du surmoulage.

En outre, il est également envisageable de réaliser dans une seule presse à injection, grâce à un moule particulier, l'injection des éléments de boîtier, l'intégration du milieu de filtration et le surmoulage en un même cycle. Il n'est alors pas nécessaire de concevoir et/ou de prévoir une machine d'assemblage adaptée au boîtier final. Cela diminue le temps de fabrication total et donc les coûts et apporte ainsi une qualité supérieure à l'unité de filtration.

Sur la figure 6, l'élément 28 est rapporté entre les deux éléments de boîtier 2,3, sans s'étendre sur la surface extérieure des éléments de boîtier 2,3.

Dans ce cas, comme illustré sur la figure 6, les deux parois latérales périphériques 25,26 formant en partie le bord périphérique 27 du boîtier présentent une surface extérieure courbe.

En relation avec la figure 7a, l'élément rapporté 28 présente un profil en U dont chaque branche s'étend sensiblement dans respectivement un plan médian P5,P6 pour être disposée dans le prolongement d'un fond 9,10.

Plus précisément, l'élément rapporté 28 s'étend en partie sur le premier et le deuxième élément de boîtier 2,3. Dans ce cas, l'élément rapporté 28 est avantageusement réalisé avec un matériau thermoplastique élastomère, de sorte à protéger l'unité de filtration des éventuels chocs et à éviter d'endommager le système à poches pendant la centrifugation. L'élément rapporté 28 possède ainsi non seulement une fonction d'assemblage mais aussi de coussin protecteur.

En outre, les périphéries des éléments de boîtier 2,3 présentent une géométrie complémentaire qui est agencée pour former un évidement périphérique 29 à leur interface, l'élément rapporté 28 étant disposé dans ledit évidement.

Notamment, la géométrie et les dimensions de l'évidement périphérique 29 sont configurés de sorte que, lors du surmoulage, l'élément rapporté 28 et/ou les éléments de boîtier 2,3 ne fondent pas au niveau du milieu de filtration 4.

L'élément rapporté 28 est réalisé dans un matériau identique ou différent de celui des éléments de boîtier 2,3. Avantageusement, pour des raisons de compatibilité et d'accroche chimique, l'élément rapporté 28 est réalisé dans un matériau polymère thermoplastique de la même famille que le matériau utilisé pour les éléments 2,3 de boîtier.

Notamment, l'élément rapporté 28 est réalisé à base d'un matériau thermoplastique, notamment un matériau élastomère thermoplastique.

Par exemple, l'élément rapporté 28 est réalisé dans un matériau polymère thermoplastique tel que le polycarbonate, polychlorure de vinyle, polypropylène ou acrylonitrile-butadiène-styrène.

En variante, l'élément rapporté 28 est réalisé dans un matériau élastomère thermoplastique, notamment à base de polyuréthane ou à base de polymères styréniques. Des exemples de matériau de surmoulage sont les produits Dryflex de la société Hexpol ou Hytrel de la société DuPont.

En particulier, l'élément rapporté 28 possède une dureté comprise entre 50 et 100 shore A, inférieure à la dureté d'au moins l'un des éléments de boîtier 2,3. Par exemple, l'élément d'étanchéité possède une dureté d'environ 70 shore A.

Selon une deuxième réalisation, les périphéries des éléments de boîtier 2, 3 présentent une géométrie complémentaire qui est agencée pour former le bord périphérique 27 ayant un profil extérieur courbe.

Notamment, en relation avec la figure 7b, chacun des éléments de boîtier 2,3 comprend un élément intérieur de conjugaison 49,50 sensiblement parallèle à la paroi latérale 25,26. Les deux éléments intérieurs de conjugaison 49,50 de chacun des éléments de boîtier 2,3 coopèrent par conjugaison de forme, de sorte qu'une fois les éléments de boîtier 2,3 assemblés entre eux, une partie creuse 51 est formée entre les parois latérales 25,26 et éléments intérieurs de conjugaison 49,50.

En variante non représentée, le boîtier de l'unité de filtration 1 comprend en outre un ou plusieurs éléments protecteurs rapportés par surmoulage d'un matériau thermoplastique élastomère configurés pour protéger le boîtier des chocs et/ou éviter d'endommager les poches souples.

Le milieu de filtration 4 est disposé entre les deux éléments de boîtier 2,3 et y est maintenu serré.

Dans une réalisation particulière et afin d'assurer une étanchéité au fluide entre le compartiment d'entrée 5 et le compartiment de sortie 6 de l'unité de filtration 1, chacun des éléments de boîtier 2,3 comprend une saillie intérieure 30,31 agencée pour comprimer le milieu de filtration 4 le long de sa périphérie.

Les saillies intérieures 30,31 sont agencées pour comprimer le milieu de filtration 4 le long de sa périphérie de sorte à créer une zone intérieure d'étanchéité au fluide.

Avantageusement, chacun des éléments de boîtier 2,3 comprend en outre une saillie extérieure 32,33 agencée pour comprimer le milieu de filtration 4 le long de sa périphérie.

Les saillies extérieures 32,33 sont agencées pour comprimer le milieu de filtration 4 de sorte à créer une zone extérieure d'étanchéité au fluide.

Dans la description et en relation avec l'unité de filtration, l'adjectif "extérieur" est à comprendre par opposition à l'adjectif "intérieur". Un moyen dit "intérieur" désigne un moyen situé dans une zone de l'unité de filtration plus proche du centre de l'unité de filtration qu'un moyen dit "extérieur".

Ainsi, si le fluide n'est pas arrêté ou suffisamment ralenti par le premier couple des saillies intérieures 30,31, le couple des saillies extérieures 32,33 fournit un deuxième passage rétréci, suffisant pour éviter le contournement du milieu de filtration 4 par le fluide.

Ceci peut être le cas si, par exemple, un défaut ou une irrégularité dans le milieu de filtration 4 se situe au niveau des saillies intérieures 30,31, empêchant le milieu de filtration 4 d'être suffisamment comprimé pour créer une étanchéité au fluide.

Par exemple, sur la figure 6, les saillies intérieures 30,31 et extérieures 32,33 sont formées de nervures continues. Ces nervures présentent une section de forme essentiellement triangulaire, à sommet arrondi.

En relation avec les éléments de boîtier 2,3, ceux-ci comprennent notamment une première et une deuxième partie de serrage 34,35, respectivement agencées pour maintenir serré dans le boîtier ledit milieu de filtration le long de sa périphérie.

Les parties de serrage 34,35 des éléments de boîtier compriment le milieu de filtration 4 de manière à en réduire l'épaisseur, sans pour autant pouvoir assurer une étanchéité au fluide.

Notamment, les parties de serrage 34,35 sont agencées sur le fond 9,10 des cavités des éléments de boîtiers 2,3.

Plus particulièrement et comme représenté sur les figures 7a et 7b, le premier et le deuxième élément de boîtier 2,3 comprennent respectivement une première et une deuxième cavité pourvues chacune d'une plate-forme périphérique dirigée vers l'extérieur formant la première et deuxième partie de serrage 34,35 respectivement, agencées pour maintenir serré dans le boîtier le milieu de filtration 4 le long de sa périphérie.

Dans cette configuration, le milieu de filtration 4 est maintenu serré entre la première et la deuxième plate-forme, ce qui permet de compresser le milieu de filtration le long de sa périphérie. Les saillies intérieures 30,31 et extérieures 32,33 sont alors situées sur ces plates-formes, notamment à leur extrémité intérieure.

Selon une forme particulière de réalisation, le premier élément de boîtier 2 et le deuxième élément de boîtier 3 comprennent chacun sur leur surface intérieure, en particulier sur la surface intérieure du fond 9,10, un déflecteur de flux formé d'une pluralité de reliefs 36,37.

En relation avec la figure 6, chacun des éléments de boîtier 2,3 est pourvu sur la surface intérieure du fond 9,10 d'une pluralité de reliefs 36,37 linéaires dirigés obliquement vers le conduit d'entrée et de sortie. Du côté du compartiment d'entrée 5, les déflecteurs aident à répartir le flux de fluide à filtrer sur toute la surface du milieu de filtration. Du côté du compartiment de sortie 6, les déflecteurs aident à diriger le fluide filtré vers le conduit de sortie de l'unité de filtration.

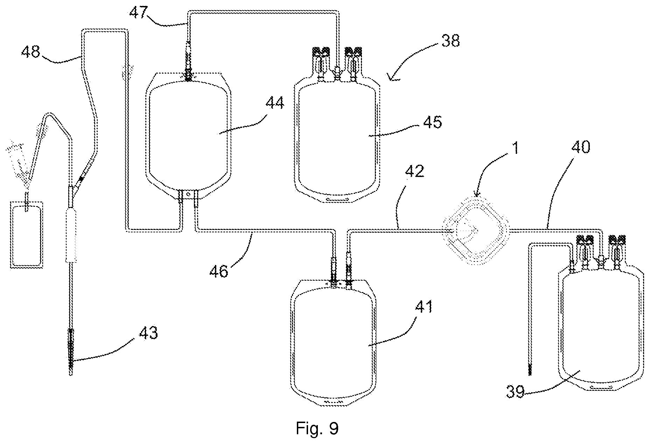

Selon un deuxième aspect et en relation avec la figure 9, l'invention concerne un système à poches 38 pour la filtration d'un fluide, comprenant :
- une unité de filtration 1 selon le premier aspect de l'invention, et
- une poche de recueil du filtrat 39, ladite poche de recueil du filtrat 39 étant reliée, par l'intermédiaire d'une première tubulure 40, au conduit de sortie 8 de l'unité de filtration 1.

Pour introduire le fluide à filtrer, le système à poches 38 comprend en outre une poche 41 destinée à contenir le fluide à filtrer, ladite poche 41 pour le fluide à filtrer étant reliée par l'intermédiaire d'une deuxième tubulure 42, au conduit d'entrée 7 de l'unité de filtration 1.

Cette poche 41 pour le fluide à filtrer est soit connectée de fabrication à l'unité de filtration 1 par l'intermédiaire de la deuxième tubulure 42, soit connectée par l'utilisateur à l'aide d'un connecteur de type perforateur pourvue à l'extrémité libre de la deuxième tubulure (non représentée).

La figure 9 représente plus particulièrement un exemple d'un système à poches 38 utilisé pour séparer en plasma et concentré de globules rouges un don de sang total et pour déleucocyter le concentré de globules rouges.

Le système à poches 38 comprend une aiguille 43 pour prélever le sang d'un donneur, une poche primaire 44 destinée à recueillir du sang total, une poche satellite 45 destinée à recevoir le plasma en communication fluidique avec la poche primaire, une poche satellite 41 destinée à recevoir le concentré de globules rouges en communication fluidique avec la poche primaire, une unité de filtration 1 telle que décrite précédemment destinée à filtrer les leucocytes du concentré de globules rouges en communication fluidique avec la poche satellite pour recevoir le concentré de globules rouges, et une poche de recueil du filtrat 39 en communication fluidique avec l'unité de filtration 1. Les poches et l'unité de filtration 1 sont reliées entre elles au moyen de tubulures souples 40,42,46,47,48 et stérilisables.

La poche primaire 44 contient un anticoagulant de type CPD et la poche de recueil du filtrat 39 contient une solution additive pour la conservation des globules rouges de type SAGM (Saline Adénine Glucose Mannitol).

En utilisation, le système à poches 38 contenant le sang total mélangé avec la solution anticoagulante dans la poche primaire 44 est placé dans un godet de centrifugation pour être centrifugé de sorte à séparer le sang en trois couches : un surnageant comprenant le plasma, une couche intermédiaire dite buffy coat comprenant un mélange de plaquettes, plasma et globules rouges et un culot comprenant le concentré de globules rouges.

Par pression sur la poche primaire 44, le plasma est envoyé dans la poche satellite 45 destinée à recevoir le plasma et le concentré de globules rouges est envoyé dans la poche satellite 41 destinée à recevoir les globules rouges. Le buffy-coat reste dans la poche primaire 44.

La solution additive est ensuite envoyée de la poche 39 de recueil du filtrat dans la poche satellite 41 destinée à recevoir les globules rouges. Le concentré de globules rouges, mélangé avec la solution additive, est finalement filtré par gravité au travers de l'unité de filtration 1 et le filtrat est reçu dans la poche destinée à recueillir le filtrat 39.

## Revendications

1. Unité de filtration (1) destinée à la filtration d'un fluide comprenant un boîtier formé d'un premier élément de boîtier (2) et d'un deuxième élément de boîtier (3) entre lesquels un milieu de filtration (4) est disposé de sorte à définir un compartiment d'entrée (5) pour le fluide à filtrer et un compartiment de sortie (6) pour le fluide filtré, ledit boîtier comportant un conduit d'entrée (7) débouchant dans le compartiment d'entrée (5) et un conduit de sortie (8) débouchant dans le compartiment de sortie (6), chacun desdits éléments de boîtier comprenant un fond (9,10) s'étendant dans un plan médian (P5,P6) en étant entouré par une périphérie, lesdits éléments de boîtier (2,3) étant assemblés entre eux de façon étanche au niveau de leur périphérie, ladite unité de filtration étant **caractérisée en ce que** le boîtier présente un bord périphérique (27) ayant un profil extérieur courbe qui s'étend entre les plans médians (P5,P6) des fonds.

2. Unité de filtration selon la revendication 1, **caractérisée en ce que** le profil extérieur courbe du bord périphérique (27) présente un rayon de courbure qui est supérieur à la moitié de la distance entre les deux plans médians (P5,P6).

3. Unité de filtration selon l'une des revendications 1 ou 2, **caractérisée en ce que** les périphéries des éléments de boîtier (2,3) présentent une géométrie complémentaire qui est agencée pour former le bord périphérique (27) ayant un profil extérieur courbe.

4. Unité de filtration selon l'une des revendications 1 ou 2, **caractérisée en ce qu'**elle comprend en outre un élément (28) qui est rapporté sur l'assemblage des périphéries des éléments de boîtier (2,3), le bord périphérique (27) ayant un profil extérieur courbe étant formé sur ledit élément rapporté (28).

5. Unité de filtration selon la revendications 4, **caractérisée en ce que** l'élément (28) est rapporté par surmoulage sur l'assemblage des périphéries des éléments de boîtier (2,3).

6. Unité de filtration selon l'une des revendications 4 ou 5, **caractérisée en ce que** l'élément rapporté (28) présente un profil en U dont chaque branche s'étend sensiblement dans respectivement un plan médian (P5,P6) pour être disposée dans le prolongement d'un fond (9,10).

7. Unité de filtration selon l'une quelconque des revendications 4 à 6, **caractérisée en ce que** les périphéries des éléments de boîtier (2,3) présentent une géométrie complémentaire qui est agencée pour former un évidement périphérique (29) à leur interface, l'élément rapporté (28) étant disposé dans ledit évidement (29).

8. Unité de filtration selon l'une quelconque des revendications 4 à 7, **caractérisée en ce que** l'élément rapporté (28) est réalisé à base d'un matériau thermoplastique, notamment un matériau élastomère thermoplastique.

9. Unité de filtration selon l'une quelconque des revendications 4 à 8, **caractérisée en ce que** l'élément rapporté (28) possède une dureté comprise entre 50 et 100 shore A.

10. Unité de filtration selon l'une des quelconque des revendications 1 à 9, **caractérisée en ce que** le fond (9,10) d'au moins un des éléments de boîtier (2,3) comprend une portion principale (11,12) et au moins une portion en dôme (13,14) qui est délimitée par un pourtour en présentant une paroi interne (15,16) s'étendant depuis ledit pourtour vers un apex (17,18) et une paroi externe (19,20) s'étendant depuis ledit apex (17,18) vers ledit pourtour, au moins un des conduits d'entrée et sortie (7,8) débouchant au travers de la paroi externe (19,20), les parois interne et externe s'étendant respectivement dans un plan médian (P1,P2,P3,P4,P5) formant entre eux un angle strictement supérieur à 90°, l'apex (17,18) présentant un profil extérieur courbe qui s'étend entre lesdits plans médians à la jonction entre les parois interne (15,16) et externe (19,20).

11. Unité de filtration selon l'une quelconque des revendications 1 à 10, **caractérisée en ce que** le conduit d'entrée (7) et le conduit de sortie (8) sont agencés parallèlement au milieu de filtration (4).

12. Unité de filtration selon l'une quelconque des revendications 1 à 11, **caractérisée en ce que** l'un au moins des conduits d'entrée et de sortie (7,8) présente à son extrémité interne (23,24) une ouverture ayant une section plus petite que la section de l'ouverture dudit conduit à son extrémité externe (21,22).

13. Unité de filtration selon l'une quelconque des revendications 1 à 12, **caractérisée en ce que** chacun des éléments de boîtier (2,3) comprend une saillie intérieure (30,31) agencée pour comprimer le milieu de filtration (4) le long de sa périphérie, ou une saillie intérieure (30,31) et une saillie extérieure (32,33) agencées pour comprimer le milieu de filtration (4) le long de sa périphérie.

14. Unité de filtration selon l'une quelconque des revendications 1 à 13, **caractérisée en ce que** le premier élément de boîtier (2) et le deuxième élément de boîtier (3) comprennent chacun sur leur surface intérieure un déflecteur de flux formé d'une pluralité de reliefs (36,37).

15. Unité de filtration selon l'une quelconque des revendications 1 à 14, **caractérisée en ce que** le milieu de filtration (4) comprend un assemblage de couches de non-tissé, notamment apte à éliminer les leucocytes du sang ou d'un composant sanguin.

16. Système à poches (38) pour la filtration d'un fluide, **caractérisé en ce qu'**il comprend :
- une unité de filtration selon l'une quelconque des revendications 1 à 15, et
- une poche de recueil du filtrat (39), ladite poche de recueil (39) étant reliée, par l'intermédiaire d'une première tubulure (40), au conduit de sortie (8) de l'unité de filtration (1).

## Patentansprüche

1. Filtrationseinheit (1), die dazu bestimmt ist, ein Fluid zu filtrieren, umfassend ein Gehäuse, das aus einem ersten Gehäuseelement (2) und einem zweiten Gehäuseelement (3) gebildet ist, zwischen denen ein Filtrationsmedium (4) angeordnet ist, um eine Einlasskammer (5) für das zu filtrierende Fluid und eine Auslasskammer (6) für das filtrierte Fluid zu definieren, wobei das Gehäuse eine Einlassleitung (7), die in die Einlasskammer (5) mündet, und eine Auslassleitung (8) umfasst, die in die Auslasskammer (6) mündet, wobei jedes der Gehäuseelemente einen Boden (9, 10) umfasst, der sich in einer Mittelebene (P5, P6) erstreckt und von einem Umfang umgeben ist, wobei die Gehäuseelemente (2, 3) an ihrem Umfang auf abgedichtete Weise zusammengebaut sind, wobei die Filtrationseinheit **dadurch gekennzeichnet ist, dass** das Gehäuse einen Umfangsrand (27) mit einem gekrümmten Außenprofil aufweist, der sich zwischen den Mittelebenen (P5, P6) der Böden erstreckt.

2. Filtrationseinheit nach Anspruch 1, **dadurch gekennzeichnet, dass** das gekrümmte Außenprofil des Umfangsrands (27) einen Krümmungsradius aufweist, der größer ist als die Hälfte des Abstands zwischen den beiden Mittelebenen (P5, P6).

3. Filtrationseinheit nach einem der Ansprüche 1 oder 2, **dadurch gekennzeichnet, dass** die Umfänge der Gehäuseelemente (2, 3) eine komplementäre Geometrie aufweisen, die dazu angeordnet ist, den Umfangsrand (27) mit einem gekrümmten Außenprofil zu bilden.

4. Filtrationseinheit nach einem der Ansprüche 1 oder 2, **dadurch gekennzeichnet, dass** sie ferner ein Element (28) umfasst, das beim Zusammenbauen der Umfänge der Gehäuseelemente (2, 3) aufgebracht wird, wobei der Umfangsrand (27) mit einem gekrümmten Außenprofil auf dem aufgebrachten Element (28) ausgebildet wird.

5. Filtrationseinheit nach Anspruch 4, **dadurch gekennzeichnet, dass** das Element (28) beim Zusammenbauen der Umfänge der Gehäuseelemente (2, 3) durch Umspritzen aufgebracht wird.

6. Filtrationseinheit nach einem der Ansprüche 4 oder 5, **dadurch gekennzeichnet, dass** das aufgebrachte Element (28) ein U-förmiges Profil aufweist, dessen Schenkel sich jeweils im Wesentlichen in einer Mittelebene (P5, P6) erstreckt, um in der Verlängerung eines Bodens (9, 10) angeordnet zu werden.

7. Filtrationseinheit nach einem der Ansprüche 4 bis 6, **dadurch gekennzeichnet, dass** die Umfänge der Gehäuseelemente (2, 3) eine komplementäre Geometrie aufweisen, die dazu angeordnet ist, an ihrer Schnittstelle eine umlaufende Aussparung (29) zu bilden, wobei das aufgebrachte Element (28) in der Aussparung (29) angeordnet ist.

8. Filtrationseinheit nach einem der Ansprüche 4 bis 7, **dadurch gekennzeichnet, dass** das aufgebrachte Element (28) auf Basis eines thermoplastischen Materials, insbesondere eines thermoplastischen Elastomermaterials, hergestellt wird.

9. Filtrationseinheit nach einem der Ansprüche 4 bis 8, **dadurch gekennzeichnet, dass** das aufgebrachte Element (28) eine Härte zwischen 50 und 100 Shore A besitzt.

10. Filtrationseinheit nach einem der Ansprüche 1 bis 9, **dadurch gekennzeichnet, dass** der Boden (9, 10) des mindestens einen der Gehäuseelemente (2, 3) einen Hauptabschnitt (11, 12) und mindestens einen Kuppelabschnitt (13, 14) umfasst, der durch eine Umrandung begrenzt ist, indem er eine Innenwand (15, 16), die sich von der Umrandung zu einem Scheitelpunkt (17, 18) erstreckt, und eine Außenwand (19, 20) aufweist, die sich von dem Scheitelpunkt (17, 18) in Richtung der Umrandung erstreckt, wobei mindestens eine der Einlassleitung und der Auslassleitung (7, 8) in die Außenwand (19, 20) mündet, wobei sich die Innenwand und die Außenwand jeweils in einer Mittelebene (P1, P2, P3, P4, P5) erstrecken, die zwischen sich einen Winkel bilden, der strikt größer als 90° ist, wobei der Scheitelpunkt (17, 18) ein gekrümmtes Außenprofil aufweist, das sich zwischen den Mittelebenen an der Verbindung zwischen der Innenwand (15, 16) und der Außenwand (19, 20) erstreckt.

11. Filtrationseinheit nach einem der Ansprüche 1 bis 10, **dadurch gekennzeichnet, dass** die Einlassleitung (7) und die Auslassleitung (8) parallel zum Filtrationsmedium (4) angeordnet sind.

12. Filtrationseinheit nach einem der Ansprüche 1 bis 11, **dadurch gekennzeichnet, dass** die mindestens eine der Einlassleitung und der Auslassleitung (7, 8) an ihrem inneren Ende (23, 24) eine Öffnung aufweist, die einen kleineren Querschnitt als der Querschnitt der Öffnung der Leitung an ihrem äußeren Ende (21, 22) aufweist.

13. Filtrationseinheit nach einem der Ansprüche 1 bis 12, **dadurch gekennzeichnet, dass** jedes der Gehäuseelemente (2, 3) einen inneren Vorsprung (30, 31), der dazu angeordnet ist, das Filtermedium (4) entlang seines Umfangs zu komprimieren, oder einen inneren Vorsprung (30, 31) und einen äußeren Vorsprung (32, 33) umfasst, die dazu angeordnet sind, das Filtermedium (4) entlang seines Umfangs zu komprimieren.

14. Filtrationseinheit nach einem der Ansprüche 1 bis 13, **dadurch gekennzeichnet, dass** das erste Gehäuseelement (2) und das zweite Gehäuseelement (3) an ihrer Innenfläche jeweils einen Strömungsablenker umfassen, der aus einer Vielzahl von Reliefs (36, 37) gebildet ist.

15. Filtrationseinheit nach einem der Ansprüche 1 bis 14, **dadurch gekennzeichnet, dass** das Filtrationsmedium (4) eine Anordnung von Vliesschichten umfasst, die insbesondere dazu geeignet ist, Leukozyten aus dem Blut oder einem Blutbestandteil zu eliminieren.

16. Beutelsystem (38) zur Filtration eines Fluids, **dadurch gekennzeichnet, dass** es Folgendes umfasst:
- eine Filtrationseinheit nach einem der Ansprüche 1 bis 15, und
- einen Beutel zum Sammeln des Filtrats (39), wobei der Sammelbeutel (39) über einen ersten Schlauch (40) mit der Auslassleitung (8) der Filtrationseinheit (1) verbunden ist.

## Claims

1. A filtration unit (1) for filtering a fluid comprising a housing formed by a first housing element (2) and a second housing element (3) between which a filtration medium (4) is disposed so as to define an inlet compartment (5) for the fluid to be filtered and an outlet compartment (6) for the filtered fluid, said housing having an inlet duct (7) opening into the inlet compartment (5) and an outlet duct (8) opening into the outlet compartment (6), each of said housing elements comprising a bottom (9, 10) extending in a median plane (P5, P6) and surrounded by a periphery, said housing elements (2, 3) being sealingly connected to each other at their periphery, said filtration unit being **characterized in that** the housing has a peripheral edge (27) with a curved outer profile extending between the median planes (P5, P6) of the bottoms.

2. Filtration unit according to claim 1, **characterized in that** the curved outer profile of the peripheral edge (27) has a radius of curvature which is greater than half the distance between the two median planes (P5, P6).

3. Filtration unit according to one of claims 1 or 2, **characterized in that** the peripheries of the housing elements (2, 3) have a complementary geometry which is arranged to form the peripheral edge (27) having a curved outer profile.

4. Filtration unit according to one of claims 1 or 2, **characterized in that** it further comprises an element (28) which is attached to the assembly of the peripheries of the housing elements (2, 3), the peripheral edge (27) having a curved outer profile being formed on said attached element (28).

5. Filtration unit according to claim 4, **characterized in that** the element (28) is added by overmolding on the assembly of the peripheries of the housing elements (2, 3).

6. Filtration unit according to one of claims 4 or 5, **characterized in that** the attached element (28) has a U-shaped profile, each leg of which extends substantially in a respective median plane (P5, P6) to be arranged in the extension of a bottom (9, 10).

7. Filtration unit according to any one of claims 4 to 6, **characterized in that** the peripheries of the housing elements (2, 3) have a complementary geometry which is arranged to form a peripheral recess (29) at their interface, the attached element (28) being disposed in said recess (29).

8. Filtration unit according to any one of claims 4 to 7, **characterized in that** the attached element (28) is made from a thermoplastic material, in particular a thermoplastic elastomer material.

9. Filtration unit according to any one of claims 4 to 8, **characterized in that** the attached element (28) has a hardness of between 50 and 100 shore A.

10. Filtration unit according to any one of claims 1 to 9, **characterized in that** the bottom (9, 10) of at least one of the housing elements (2, 3) comprises a main portion (11, 12) and at least one domed portion (13, 14) which is delimited by a perimeter while having an inner wall (15, 16) extending from said perimeter towards an apex (17, 18) and an outer wall (19, 20) extending from said apex (17,18) towards said perimeter, at least one of the inlet and outlet ducts (7, 8) opening through the outer wall (19, 20), the inner and outer walls extending respectively in a median plane (P1, P2, P3, P4, P5) forming between them an angle strictly greater than 90°, the apex (17, 18) having a curved outer profile which extends between said median planes at the junction between the inner (15, 16) and outer (19, 20) walls.

11. Filtration unit according to any one of claims 1 to 10, **characterized in that** the inlet duct (7) and the outlet duct (8) are arranged parallel to the filtration medium (4).

12. Filtration unit according to any one of claims 1 to 11, **characterized in that** at least one of the inlet and outlet ducts (7, 8) has an opening at its inner end (23, 24) with a cross-section smaller than the cross-section of the opening of said duct at its outer end (21, 22).

13. A filtration unit according to any one of claims 1 to 12, **characterized in that** each of the housing elements (2, 3) comprises an inner projection (30, 31) arranged to compress the filtration medium (4) along its periphery, or an inner projection (30, 31) and an outer projection (32, 33) arranged to compress the filtration medium (4) along its periphery.

14. Filtration unit according to any one of claims 1 to 13, **characterized in that** the first housing element (2) and the second housing element (3) each comprise on their inner surface a flow deflector formed by a plurality of reliefs (36, 37).

15. Filtration unit according to any one of claims 1 to 14, **characterized in that** the filtration medium (4) comprises an assembly of non-woven layers, particularly suitable for removing leukocytes from blood or a blood component.

16. A bag system (38) for filtering a fluid, **characterized in that** it comprises:
- a filtration unit according to any of claims 1 to 15, and
- a filtrate collection bag (39), said collection bag (39) being connected, via a first tube (40), to the outlet duct (8) of the filtration unit (1).
